# EUROPÄISCHE PATENTSCHRIFT

(11) **EP 1 372 624 B1**
(45) Veröffentlichungstag und Bekanntmachung des Hinweises auf die Patenterteilung: **24.08.2005**
(21) Anmeldenummer: 02722102.7
(22) Anmeldetag: 20.02.2002
(51) Int. Cl.: A61K 31/135, A61K 9/22, A61K 9/52, A61P 29/00, A61P 13/10, A61P 11/14, A61P 1/12, A61P 25/24, A61P 25/00

(54) **ARZNEIMITTEL AUF BASIS VON TRAMADOL**
TRAMADOL-BASED MEDICAMENT
MEDICAMENT A BASE DE TRAMADOL

(30) Priorität: 21.02.2001 DE 10108122
(43) Veröffentlichungstag der Anmeldung: 02.01.2004
(73) Patentinhaber: Grünenthal GmbH, 52078 Aachen (DE)
(72) Erfinder: BARTHOLOMÄUS, Johannes, 52080 Aachen (DE); FRIDERICHS, Elmar, 52223 Stolberg (DE)
(74) Vertreter: Kutzenberger, Helga
(86) Internationale Anmeldenummer: PCT/EP2002/001763
(87) Internationale Veröffentlichungsnummer: WO 2002/066026

(56) Entgegenhaltungen:
- EP-A- 0 642 788
- EP-A- 1 005 861
- WO-A-00/32558
- GB-A- 2 317 110
- US-A- 5 516 803
- GROND S ET AL: "ANALGESIC EFFICACY AND SAFETY OF TRAMADOL ENANTIOMERS IN COMPARISON WITH THE RACEMATE: A RANDOMISED, DOUBLE-BLIND STUDY WITH GYNAECOLOGICAL PATIENTS USING INTRAVENOUS PATIENT-CONTROLLED ANALGESIA" PAIN, ELSEVIER SCIENCE PUBLISHERS, AMSTERDAM, NL, Bd. 62, Nr. 3, September 1995 (1995-09), Seiten 313-320, XP000891573 ISSN: 0304-3959
- ROJAS-CORRALES M O ET AL: "TRAMADOL INDUCES ANTIDEPRESSANT-TYPE EFFECTS IN MICE" LIFE SCIENCES, PERGAMON PRESS, OXFORD, GB, Bd. 63, Nr. 12, 1998, Seiten 175-180, XP000910402 ISSN: 0024-3205

## Beschreibung

Die vorliegende Erfindung betrifft ein Arzneimittel enthaltend das Racemat des Tramadols in retardierter Form und das (+)-Enantiomer des Tramadols in unretardierter Form.

Der pharmazeutische Wirkstoff Tramadol wird üblicherweise in Form seines Racemates aus (+)-Tramadol - d.h. (1R,2R)-2-[(dimethylamino)methyl]-1-(3-methoxyphenyl)-cyclohexanol und (-)-Tramadol - d.h. (1S,2S)-2-[(dimethylamino)methyl]-1-(3-methoxyphenyl)-cyclohexanol - zur Bekämpfung mittelstarker bis starker Schmerzen eingesetzt. Beim Überschreiten einer gewissen Schmerzintensität ist die analgetische Wirksamkeit des Tramadols für eine zufriedenstellende Schmerztherapie des Patienten allerdings häufig nicht mehr ausreichend.

Die analgetische Wirksamkeit des Tramadols beruht auf einem komplizierten Zusammenspiel seiner Enantiomeren nach einem Mechanismus, der neben einer opioiden auch eine nicht-opioide Wirkkomponente umfaßt. Die opioide Wirkkomponente, die den wesentlichen Beitrag zur analgetischen Wirksamkeit des Tramadols leistet, ist auf das (+)-Enantiomer des Tramadols bzw. den entsprechenden Metaboliten, das (+)-O-Desmethyltramadol, zurückzuführen.

Sowohl die metabolische Aktivierung von (+)-Tramadol als auch die von (-)-Tramadol erfolgt durch das Enzym CYP2D6, das im Körper des Patienten nicht unbegrenzt zur Verfügung steht. Eine Verbesserung der analgetischen Wirksamkeit des Tramadols ab einer gewissen Schmerzgrenze bzw. unmittelbar nach der Gabe an den Patienten ist daher über eine Erhöhung der Dosierung von racemischem Tramadol nicht in ausreichenden Maße zu erzielen.

Eine Aufgabe der vorliegenden Erfindung war es daher, ein Arzneimittel auf Basis von Tramadol zur Verfügung zu stellen, das auch geeignet ist, sehr starke Schmerzen, insbesondere in der Anfangsphase der Schmerzbekämpfung, vollständig zu unterdrücken oder zumindest deutlich zu lindem.

Erfindungsgemäß wird diese Aufgabe durch die Bereitstellung eines Arzneimittels gelöst, das das Racemat des Tramadols in retardierter Form und das (+)-Enantiomer des Tramadols in unretardierter Form enthält.

Die Herstellung und gegebenenfalls die Aufreinigung und/oder Isolierung des (+)-Tramadols kann nach üblichen, dem Fachmann bekannten Methoden erfolgen, wie z.B. in Frankus et al, Arzneim.-Forschung. Drug Res. 28, Seiten 114-121, 1978 oder in der EP 0 787715 B1 beschrieben. Das Racemat des Tramadols ist allgemein am Markt erhältlich.

Das erfindungsgemäße Arzneimittel kann auch wenigstens eine dieser Wirkstoffkomponenten, das Racemat bzw. das (+)-Enantiomer des Tramadols, in Form wenigstens eines entsprechenden physiologisch verträglichen Salzes enthalten.

Vorzugsweise sind diese physiologisch verträglichen Salze ausgewählt aus der Gruppe Chlorid, Bromid, Sulfat, Sulfonat, Phosphat, Tartrat, Teoclat, Embonat, Formiat, Acetat, Propionat, Benzoat, Oxalat, Succinat, Citrat, Diclofenacat, Naproxenat, Salicylat, Acetylsalicylat, Glutamat, Fumarat, Aspartat, Glutarat, Stearat, Butyrat, Malonat, Lactat, Mesylat, Saccharinat, Cyclamat und Acesulfamat, besonders bevorzugt aus der Gruppe Chlorid, Sulfat, Saccharinat, Teoclat, Embonat, Diclofenacat, Naproxenat und Salicylat. Ganz besonders bevorzugt liegt als Salz der jeweiligen Wirkstoffkomponente das entsprechende Chlorid vor.

Die physiologisch verträglichen Salze bzw. Säureadditionssalze lassen sich nach den üblichen, dem Fachmann bekannten Methoden, beispielsweise durch die Umsetzung des Tramadol-Racemates oder des (+)-Tramadols mit der entsprechenden Säure, vorzugsweise in wäßriger Lösung, erhalten.

Vorzugsweise enthält das erfindungsgemäße Arzneimittel 10 bis 75 Gew.-% (+)-Tramadol und 90 bis 25 Gew.-% retardiertes racemisches Tramadol, besonders bevorzugt 20 bis 50 Gew.-% (+)-Tramadol und 80 bis 50 Gew.-% des retardierten racemischen Tramadols, wobei diese Mengen jeweils als Wirkstoff und nicht als Wirkstoffsalz berechnet und auf die Gesamtmenge an Wirkstoffen bezogen sind.

Das erfindungsgemäße Arzneimittel eignet sich bevorzugt zur parenteralen oder oralen, besonders bevorzugt zur oralen Applikation. Unter oral applizierbaren Arzneimitteln werden dabei erfindungsgemäß sowohl solche Arzneimittel verstanden, die im Mundbereich resorbiert werden, als auch solche, die über den Mund eingenommen, aber erst im Magen-Darm-Trakt resorbiert werden.

In einer bevorzugten Formulierungsform liegt das erfindungsgemäße Arzneimittel in Form von Sirupen, transmucosalen therapeutischen Systemen, transdermalen therapeutischen Systemen, Suspensionen, Tabletten, Mehrschichttabletten, Dragees, Kapseln, Suppositorien, leicht rekonstituierbaren Trockenzubereitungen oder Pulvern vor. In einer besonders bevorzugten Ausführungsform liegt das erfindungsgemäße Arzneimittel in Form von Tabletten, Mehrschichttabletten, Kapseln oder als Suspension vor.

In einer besonders bevorzugten Ausführungsform weist das erfindungsgemäße Arzneimittel das retardierte Racemat des Tramadols und das (+)-Enantiomer des Tramadols in jeweils voneinander separiert formulierten Untereinheiten auf.

Untereinheiten im Sinne der vorliegenden Erfindung sind feste Formulierungen, die neben der jeweiligen Wirkstoffkomponente auch physiologisch verträgliche-Hilfsstoffe enthalten können.

Vorzugsweise liegen die Untereinheiten des erfindungsgemäßen Arzneimittels in multipartikulärer Form vor. Bevorzugte multipartikuläre Untereinheiten sind Mikrotabletten, Mikrokapseln, Granulate, Wirkstoffkristalle oder Pellets. Besonders bevorzugt liegen die multipartikulären Untereinheiten in Form von Mikrotabletten, Granulaten oder Pellets vor.

Die Formulierung der multipartikulären Formen zu dem erfindungsgemäßen Arzneimittel kann nach üblichen, dem Fachmann bekannten Methoden, beispielsweise durch Abfüllen in Kapseln oder Sachets, Verpressen zu Tabletten oder durch Suspendierung in hydrophiler oder lipophiler Flüssigkeit, erfolgen.
Sofern das erfindungsgemäße Arzneimittel in Form einer Mehrschichttablette vorliegt, können die Untereinheiten verschiedene Schichten einer Mehrschichttablette, bevorzugt die Schichten einer Zweischichttablette sein oder die multipartikulären Untereinheiten können zu solchen Schichten verpreßt werden.

In einer weiteren bevorzugten Ausführungsform der vorliegenden Erfindung kann das erfindungsgemäße Arzneimittel das retardierte Racemat des Tramadols in Untereinheiten formuliert enthalten, die u.a. mit einem Überzug enthaltend das (+)-Tramadol ausgerüstet sind.

Neben dem Wirkstoff-haltigen Überzug und gegebenenfalls Retard-Überzug des Racemats können die Untereinheiten gegebenenfalls noch mindestens einen weiteren nicht retardierenden Überzug aufweisen, der als Schutzschicht direkt auf der Oberfläche der Untereinheiten aufgebracht ist.

Sofern es sich bei den multipartikulären Untereinheiten um Granulate oder Pellets handelt, weisen diese vorzugsweise eine Größe im Bereich von 0,1 bis 3 mm, besonders bevorzugt im Bereich von 0,5 bis 2 mm auf.
Liegen die multipartikulären Untereinheiten in Form von Mikrotabletten vor, haben diese vorzugsweise einen Durchmesser von 0,5 bis 5 mm, besonders bevorzugt 1 bis 3 mm und ganz besonders bevorzugt von 1 bis 2 mm.

Handelt es sich bei den multipartikulären Untereinheiten um Wirkstoffkristalle oder Mikrokapseln, so weisen diese bevorzugt einen Durchmesser von 10 µm bis 1 mm, besonders bevorzugt von 15 µm bis 0,5 mm auf. Ganz besonders bevorzugt beträgt der Durchmesser 30 µm bis 200 µm.

Das erfindungsgemäße Arzneimittel kann außerdem je nach Ausführungsform als weitere Bestandteile die üblichen, dem Fachmann bekannte physiologisch verträgliche Hilfsstoffe enthalten.

Sofern das erfindungsgemäße Arzneimittel in Form von Tabletten oder Mikrotabletten vorliegt, können diese als weitere physiologisch verträglichen Hilfsstoffe vorzugsweise mikrokristalline Cellulose, Celluloseether, Lactose, Stärke, Stärkederivate, Zuckeralkohole und/oder Calciumhydrogenphosphat sowie weitere übliche, dem Fachmann bekannte Bindemittel, Fließregulationsmittel und/oder Gleitmittel und gegebenenfalls Sprengmittel enthalten.

Liegt das erfindungsgemäße Arzneimittel in Form von Pellets oder Granulaten vor, können diese als weitere physiologisch verträgliche Hilfsstoffe bevorzugt mikrokristalline Cellulose, Celluloseether, Lactose, Stärke, Stärkederivate, Zuckeralkohole, Calciumhydrogenphosphat, Fettalkohole, Ester des Glycerins und/oder Fettsäureester enthalten.

Liegt das erfindungsgemäße Arzneimittel in Form von Mikrokapseln vor, so können diese je nach Art des zu ihrer Herstellung eingesetzten Verfahrens die üblichen, dem Fachmann bekannten physiologisch verträglichen Hilfsstoffe enthalten.

Liegt das erfindungsgemäße Arzneimittel in Form einer Suspension vor, so kann diese neben dem physiologisch verträglichen Suspensionsmedium weitere übliche, dem Fachmann bekannte physiologisch verträgliche Hilfsstoffe, wie z.B. pH-Regulatoren, Regulatoren zur Einstellung der Osmolalität, grenzflächenaktive Verbindungen, Viskositätsregulatoren, Puffer und/oder Konservierungsmittel enthalten.

Die Herstellung der verschiedenen Formulierungsformen des erfindungsgemäßen Arzneimittels kann nach verschiedenen, dem Fachmann bekannten Methoden erfolgen.

Sofern das erfindungsgemäße Arzneimittel in Form von Tabletten vorliegt, können diese beispielsweise durch das Verpressen der mittels Feucht-, Trocken- oder Schmelzgranulation hergestellten Granulate des Enantiomers und des entsprechend hergestellten und in geeigneter Form retardierten Granulats des Racemats gegebenenfalls mit weitereren physiologisch verträglichen Hilfsstoffen hergestellt werden. Desweiteren können die Tabletten durch Verpressen von multipartikulären ggf. überzogenen Pellets, Wirkstoffkristallen oder Mikrokapseln hergestellt werden, die für die racemische Komponente retardiert sind.

Die Formulierungen in Form von Pellets können vorzugsweise durch Extrusion und Spheronisation, durch Aufbaupelletisierung oder durch Direktpelletisierung in einem hochtourigen Mischer oder in der Rotorwirbelschicht unter gleichzeitiger oder anschließender Retardierung der racemischen Komponente hergestellt werden. Besonders bevorzugt ist die Herstellung der Pellets durch Extrusion feuchter Massen und anschließender Spheronisation. Die enantiomere Komponente wird vorzugsweise in Form eines Überzugs auf den Pellets aufgebracht.

Die Herstellung von Mikrokapseln erfolgt nach üblichen Mikroverkapselungsverfahren, wie z.B. durch Sprühtrocknung, Sprüherstarrung oder Koazervation, wobei für die racemische Komponente die gewünschte Retardierung erfolgt.

In einer bevorzugten Ausführungsform des erfindungsgemäßen Arzneimittels basiert die Retardierung des racemischen Tramadols auf einem retardierenden Überzug, auf der Einbettung in eine retardierende Matrix, auf der Anbindung an ein lonenaustauscherharz oder einer Kombination aus wenigstens zwei der vorstehend genannten Retardierungsmethoden.

Bevorzugt basiert der retardierende Überzug auf einem wasserunlöslichen, gegebenenfalls modifizierten natürlichen oder synthetischen Polymeren oder auf einem natürlichen, halbsynthetischen oder synthetischen Wachs oder Fett oder Fettalkohol oder einem Gemisch aus wenigstens zwei dieser vorstehend genannten Komponenten.

Als wasserunlösliche Polymere werden zur Herstellung eines retardierenden Überzugs vorzugsweise Poly(meth)acrylate, besonders bevorzugt Poly(C₁₋₄)-Alkyl(meth)acrylate, Poly(C₁₋₄)-dialkylamino-(C₁₋₄)-alkyl(meth)acrylate und/oder deren Copolymere, ganz besonders bevorzugt Ethylacrylat/Methylmethacrylat-Copolymere mit einem molaren Verhältnis der Monomeren von 2 : 1, Ethylacrylat/Methylmethacrylat/Trimethylammoniumethylmethacrylat-chlorid-Copolymere mit einem molaren Verhältnis der Monomeren von 1 : 2 : 0,1, Ethylacrylat/Methylmethacrylat/Trimethylammoniumethyl-methacrylatchlorid-Copolymere mit einem molaren Verhältnis der Monomeren von 1 : 2 : 0,2 oder ein Gemisch aus wenigstens zwei dieser vorstehend genannten Polymeren als Überzugsmaterial eingesetzt.

Diese Überzugsmaterialien sind als 30 Gew.%-ige wäßrige Latexdispersionen unter der Bezeichnung Eudragit RS30D®, Eudragit NE30D® bzw. Eudragit RL30D® am Markt erhältlich und werden als solche auch als Überzugsmaterial bevorzugt eingesetzt.

Ebenfalls bevorzugt können als wasserunlösliche Polymere zur Herstellung des retardierenden Überzugs in dem erfindungsgemäßen Arzneimittel Polyvinylacetate ggf. in Kombination mit weiteren Hilfsstoffen eingesetzt werden. Diese sind als wäßrige Dispersion enthaltend 27 Gew.-% Polyvinylacetat, 2,5 Gew.-% Povidon und 0,3 Gew.-% Natriumlaurylsulfat (Kollicoat SR 30 D®) am Markt erhältlich.

In einer weiteren bevorzugten Ausführungsform basieren die retardierenden Überzüge des racemischen Tramadols auf wasserunlöslichen Cellulosederivaten, vorzugsweise Alkylcellulosen, wie z.B. Ethylcellulose, oder auf Celluloseestern, wie z.B. Celluloseacetat als Überzugsmaterial. Die Überzüge aus Ethylcellulose oder Celluloseacetat werden bevorzugt aus wäßriger Pseudolatexdispersion aufgebracht. Wäßrige Ethylcellulose-Pseudolatexdispersionen werden als 30 Gew.%-ige Dispersionen (Aquacoat®) oder als 25 Gew.%-ige Dispersionen (Surelease®) am Markt geführt und werden als solche auch als Überzugsmaterial bevorzugt eingesetzt.

Als natürliche, halbsynthetische oder synthetische Wachse, Fette bzw. Fettalkohole kann der retardierende Überzug des racemischen Tramadols, vorzugsweise auf Camaubawachs, Bienenwachs, Glycerinmonostearat, Glycerinmonobehenat (Compritol ATO888®), Glycerinditripalmitostearat (Precirol ATO5®), mikrokristallinem Wachs, Cetylalkohol, Cetylstearylalkohol oder einem Gemisch aus wenigstens zwei dieser Komponenten basieren.

Sofern der retardierende Überzug auf einem wasserunlöslichen, gegebenenfalls modifizierten natürlichen und/oder synthetischen Polymeren basiert, kann die Überzugsdispersion oder Lösung neben dem entsprechendem Polymer einen üblichen, dem Fachmann bekannten, physiologisch verträglichen Weichmacher aufweisen, um die notwendige Mindestfilmtemperatur zu senken.

Geeignete Weichmacher sind beispielsweise lipophile Diester aus einer aliphatischen oder aromatischen Dicarbonsäure mit C₆-C₄₀ und einem aliphatischen Alkohol mit C₁-C₈, wie z.B. Dibutylphthalat, Diethylphthalat, Dibutylsebacat oder Diethylsebacat, hydrophile oder lipophile Ester der Zitronensäure, wie z.B. Triethylcitrat, Tributylcitrat, Acetyltributylcitrat oder Acetyltriethylcitrat, Polyalkylenglykole, wie z.B. Polyethylenglykole oder Propylenglykole, Ester des Glycerins, wie z.B. Triacetin, Myvacet® (acetylierte Mono- und Diglyceride, C₂₃H₄₄O₅ bis C₂₅H₄₇O₇), mittelkettige Triglyceride (Miglyol®), Ölsäure oder Gemische aus wenigstens zwei der vorstehend genannten Weichmacher.
Vorzugsweise enthalten wäßrige Dispersionen von Eudragit RS® und gegebenenfalls Eudragit RL® Triethylcitrat als Weichmacher.

Vorzugsweise enthält der retardierende Überzug den/die Weichmacher in Mengen von 5 bis 50 Gew-%., besonders bevorzugt 10 bis 40 Gew.-% und ganz besonders bevorzugt 10 bis 30 Gew-%, bezogen auf die Menge des eingesetzten Polymeren. In Einzelfällen, beispielsweise für Celluloseacetat können auch höhere Mengen an Weichmachern, vorzugsweise bis zu 110 Gew.-% eingesetzt werden.

Desweiteren kann der retardierende Überzug weitere übliche, dem Fachmann bekannte Hilfsstoffe, wie z.B. Gleitmittel, vorzugsweise Talkum oder Glycerinmonostearat, Farbpigmente, vorzugsweise Eisenoxide oder Titandioxid, oder Tenside, wie z.B. Tween 80® aufweisen.

Das Freisetzungsprofil des retardierten Tramadol-Racemates des erfindungsgemäßen Arzneimittels kann durch die üblichen, dem Fachmann bekannten Methoden, wie z.B. durch die Dicke des Überzugs oder durch den Einsatz weiterer Hilfsstoffe als Bestandteile des Überzugs eingestellt werden. Geeignete Hilfsstoffe sind beispielsweise hydrophile oder pH-abhängige Porenbildner, wie z.B. Natrium-Carboxymethylcellulose, Celluloseacetatphthalat, Hydroxypropylmethylcelluloseacetatsuccinat, Lactose, Polyethylenglykol oder Mannitol oder wasserlösliche Polymere, wie z.B. Polyvinylpyrrolidon oder wasserlösliche Cellulosen, vorzugsweise Hydroxypropylmethylcellulose oder Hydroxypropylcellulose.

Der retardierende Überzug kann auch unlösliche bzw. lipophile Hilfsstoffe, wie z.B. alkylisiertes Siliciumdioxid, das z.B. als Aerosil R972® am Markt geführt wird, oder Magnesiumstearat zur weiteren Verstärkung der Retardierung enthalten.

Das erfindungsgemäße Arzneimittel selbst kann auch mindestens einen nicht retardierenden Überzug aufweisen. Dies kann beispielsweise ein Überzug zur Geschmacksverbesserung oder ein magensaftresistenter Überzug sein, der sich pH-abhängig auflöst. Durch den magensaftresistenten Überzug kann erreicht werden, daß die entsprechende Formulierung des erfindungsgemäßen Arzneimittels den Magentrakt unaufgelöst passiert und die Wirkstofflcomponenten erst im Darmtrakt zur Freisetzung gelangen. Vorzugsweise löst sich der magensaftresistente Überzug bei einem pH-Wert zwischen 5 und 7,5 auf.

Der magensaftresistente Überzug basiert vorzugsweise auf Methacrylsäure/Methylmethacrylat-Copolymeren mit einem molaren Verhältnis der jeweiligen Monomeren von 1 : 1 (Eudragit L®), Methacrylsäure/Methylmethacrylat-Copolymeren mit einem molaren Verhältnis der jeweiligen Monomeren von 1 : 2 (Eudragit S®), Methacrylsäure/Ethylacrytat-Copolymeren mit einem molaren Verhältnis der jeweiligen Monomeren von 1:1 (Eudragit L30D-55®) Methacrylsäure/Methylacrylat/Methylmethacrylat-Copolymeren mit einem molaren Verhältnis der jeweiligen Monomeren von 7 : 3 : 1 (Eudragit FS®), Schellack Hydroxypropylmethylcelluloseacetatsuccinate, Celluloseacetatphthalate oder einer Mischung aus wenigstens zwei dieser Komponenten, die ggf. auch in Kombination mit den vorstehend genannten wasserunlöslichen Poly(meth)acrylaten, vorzugsweise in Kombination mit Eudragit NE30D® und/oder Eudragit RL® und/oder Eudragit RS® eingesetzt werden können.

Die retardierenden und/oder nicht retardierenden Überzüge können nach den üblichen, für den jeweiligen Überzug geeigneten, dem Fachmann bekannten Verfahren, wie z.B. durch Aufsprühen von Lösungen, Dispersionen oder Suspensionen, durch Schmelzverfahren oder durch Pulverauftragsverfahren aufgebracht werden. Die Lösungen, Dispersionen oder Suspensionen können in Form von wäßrigen oder organischen Lösungen oder Dispersionen eingesetzt werden. Dabei werden wäßrige Dispersionen bevorzugt eingesetzt. Als organische Lösungsmittel können Alkohole, beispielsweise Ethanol oder Isopropanol, Ketone, wie z.B. Aceton, Ester, beispielsweise Ethylacetat, chlorierte Kohlenwasserstoffe, wie z.B. Dichlormethan verwendet werden, wobei Alkohole und Ketone bevorzugt eingesetzt werden. Es ist auch möglich Mischungen aus wenigstens zwei der vorstehend genannten Lösungsmittel einzusetzen.

Sofern das erfindungsgemäße Arzneimittel das Racemat des Tramadols in multipartikulärer Form aufweist, wird der retardierende Überzug vorzugsweise so aufgebracht, daß man die multipartikulären Formen enthaltend das racemische Tramadol nach ihrer Herstellung mit den jeweiligen retardierenden Polymeren und gegebenenfalls physiologisch verträglichen Hilfsstoffen aus wässrigen und/oder organischen Medien, vorzugsweise aus wässrigen Medien, mit Hilfe des Wirbelschichtverfahrens überzieht und den Überzug vorzugsweise gleichzeitig bei üblichen Temperaturen in der Wirbelschicht trocknet, und gegebenenfalls, wenn nötig, tempert und/oder einen Überzug aus (+)-Tramadol aufbringt.

Vorzugsweise erfolgt die Trocknung des Überzuges für Poly(meth)acrylatüberzüge bei einer Zulufttemperatur im Bereich von 30 bis 50 °C, besonders bevorzugt im Bereich von 35 bis 45 °C.

Für Überzüge auf Cellulosebasis, wie z.B. Ethylcellulose oder Celluloseacetat, erfolgt die Trocknung bevorzugt bei einer Temperatur im Bereich von 50 bis 80 °C, besonders bevorzugt im Bereich von 55 bis 65 °C.

Wachsüberzüge können durch Schmelzüberziehen in der Wirbelschicht aufgebracht werden und bei Temperaturen unterhalb des jeweiligen Schmelzbereiches nach dem Überziehen zur vollständigen Verfestigung abgekühlt werden. Das Aufbringen von Wachsüberzügen kann auch durch Aufsprühen von deren Lösungen in organischen Lösungsmitteln erfolgen.

Zur Modifizierung des Wirkstoff-Freisetrungsprofils kann das erfindungsgemäße Arzneimittel das Racemat des Tramadols auch in einer retardierenden Matrix, vorzugsweise gleichmäßig verteilt, enthalten.

Als Matrixmaterialien können physiologisch verträgliche, hydrophile Materialien verwendet werden, welche dem Fachmann bekannt sind. Vorzugsweise werden als hydrophile Matrixmaterialien Polymere, besonders bevorzugt Celluloseether, Celluloseester und/oder Acrylharze verwendet. Ganz besonders bevorzugt werden als Matrixmaterialien Ethylcellulose, Hydroxypropylmethylcellulose, Hydroxypropylcellulose, Hydroxymethylcellulose, Poly(meth)acrylsäure und/oder deren Derivate, wie deren Salze, Amide oder Ester eingesetzt.

Ebenfalls bevorzugt sind Matrixmaterialien aus hydrophoben Materialien, wie hydrophoben Polymeren, Wachsen, Fetten, langkettigen Fettsäuren, Fettalkoholen oder entsprechenden Estern oder Ethem oder deren Gemische. Besonders bevorzugt werden als hydrophobe Materialien Mono- oder Diglyceride von C₁₂-C₃₀-Fettsäuren und/oder C₁₂-C₃₀-Fettalkohole und/oder Wachse oder deren Gemische eingesetzt.

Es ist auch möglich, Mischungen der vorstehend genannten hydrophilen und hydrophoben Materialien als retardierendes Matrixmaterial einzusetzen.

Die Herstellung des in einer retardierenden Matrix vorliegenden Racemats des Tramadols kann nach den üblichen, dem Fachmann bekannten Methoden erfolgen, wobei die Formulierung mit dem (+)-Tramadol zu den erfindungsgemäßen Arzneimitteln wir vorstehend angegeben erfolgen kann.

Die an den Patienten zu verabreichende Gesamtmenge des retardierten und nicht retardierten Tramadolwirkstoffes variiert z.B. in Abhängigkeit vom Gewicht des Patienten, von der Indikation sowie dem Schweregrad der Schmerzen bzw. der Erkrankung. Vorzugsweise wird die zu verabreichende Menge des retardierten und nicht retardierten Wirkstoffes sowie dessen Freisetzung so eingestellt, daß eine Gabe des Arzneimittels höchstens zweimal, vorzugsweise nur einmal täglich erfolgen muß und gleichzeitig eine ausreichende sofortige Wirkung nach Verabreichung eintritt.

Vorzugsweise eignet sich das erfindungsgemäße Arzneimittel zur Bekämpfung von Schmerzen oder zur Behandlung von Harninkontinenz, Husten, Depressionen, Diarrhoe oder seelischen Erkrankungen. Besonders bevorzugt wird das erfindungsgemäße Arzneimittel zur Bekämpfung von akuten oder chronischen Schmerzen eingesetzt.

Das erfindungsgemäße Arzneimittel hat den Vorteil, daß es die zufriedenstellende Bekämpfung sehr starker Schmerzen ermöglicht, wobei die Häufigkeit oder die Stärke gegebenenfalls auftretender unerwünschter Begleiterscheinungen des Tramadols, wie z.B. Übelkeit, Erbrechen, Schitzen, Mundtrockenheit, Schwindel, Krämpfe oder Benommenheit gar nicht oder nur geringfügig zunimmt. Das (+)-Tramadol steht unmittelbar nach der Applikation des Arzneimittels an den Patienten für die metabolische Aktivierung durch das Enzym CYP2D6 zu dem stark analgetisch wirksamen Metaboliten (+)-O-Desmethyltramadol zur Verfügung, so daß sich das erfindungsgemäße Arzneimittel insbesondere auch zur Bekämpfung sehr starker akuter Schmerzen eignet.

Bei der Bekämpfung mittlerer und starker Schmerzen kann die an den Patienten zu verabreichende Gesamtdosis an Tramadolwirkstoff gegenüber herkömlichen Tramadolformulierungen, die neben retardiertem Tramadol-Racemat auch eine Initialdosis des racemischen Tramadols enthalten, reduziert werden, ohne daß hierdurch die analgetische Wirksamkeit des Tramadols vermindert wird. Dies hat den Vorteil, daß die gegebenenfalls auftretenden unerwünschten Begleiterscheinungen des Tramadols seltener oder in abgeschwächter Form auftreten.

Ein weiterer Vorteil des erfindungsgemäßen Arzneimittels liegt darin, daß das sehr niedrige Sucht- und Abhängigkeitspotential herkömmlicher Tramadolformulierungen erhalten bleibt, während der Beitrag der opioiden Wirkkomponente zur Schmerzbekämpfung gesteigert wird.

Die Freisetzung von (+)- und (-)-Tramadol aus dem erfindungsgemäßen Arzneimittel wurde wie folgt bestimmt

Die jeweilige Formulierung des erfindungsgemäßen Arzneimttels wurde in der Drehkörbchenapparatur oder der Blattrührerapparatur des Pharm. Eur. bei einer Temperatur des Freisetzungsmediums von 37 ± 0,5 °C bei einer Umdrehungsgeschwindigkeit von 100 Umdrehungen pro Minute im Falle der Drehkörbchenapparatur bzw. 75 Umdrehungen pro Minute im Falle der Blattrührapparatur 2 Stunden lang in 600 ml künstlichem Magensaft bei pH 1,2 ohne Enzyme geprüft. Anschließend wurde die Formulierung weitere 8 Stunden lang in 600 ml künstlichem Darmsaft bei pH 7,2 ohne Enzyme geprüft. Die jeweils zu einem Zeitpunkt freigesetzte Menge von (+)-Tramadol und (-)-Tramadol wurde mittels HPLC bestimmt. Die dargestellten Werte sind die Mittelwerte aus jeweils 6 Proben.

Im folgenden wird die Erfindung anhand von Beispielen erläutert. Diese Erläuterungen sind lediglich beispielhaft und schränken den allgemeinen Erfindungsgedanken nicht ein.

### Beispiele:

### Beispiel 1:

### Herstellung der Pellets:

Pellets enthaltend das Racemat des Tramadols mit einem Wirkstoffgehalt von 55 Gew.% wurden durch wäßrige Granulation mit mikrokristalliner Cellulose und niedrig substituierter Hydroxypropylcellulose und anschließender Extrusion und Spheronisation hergestellt. Die so erhaltenen Pellets wurden getrocknet, auf eine Größe von von 800-1250 µm abgesiebt und anschließend in der Wirbelschicht bei einer Zulufttemperatur von 60°C zuerst mit 3 Gew.% Hydroxypropylmethylcellulose und Talkum als Überzug und anschließend mit 11 Gew.% Surelease® E-7-7050 als retardierendem Überzug befilmt. Die Filmaufträge sind jeweils in Gewichtsprozent, bezogen auf das Ausgangsgewicht der Pellets bzw. der überzogenen Pellets angegeben.

Pellets enthaltend das (+)-Enantiomer des Tramadols mit einem Wirkstoffgehalt von 55 Gew.-% wurden durch wäßrige Granulation mit mikrokristalliner Cellulose und niedrig substituierter Hydroxypropylcellulose und anschließender Extrusion und Spheronisation hergestellt. Die so erhaltenen Pellets wurden getrocknet und auf eine Größe von 800 bis 1250 µm abgesiebt. Diese Pellets wurden dann mit einem nicht retardierenden Überzug aus Hydroxypropylmethylcellulose (Opadry OY 29020 clear®) überzogen.

Auf einer geeigneten Steckkapselmaschine wurden dann Hartgelatinekapseln der Größe 1 mit 212 mg retardierten Pellets enthaltend racemisches Tramadol (entsprechend 100 mg racemischem Tramadolhydrochlorid) und 47 mg der unretardierten Pellets enthaltend das (+)-Enantiomer des Tramadols (entsprechend 25 mg (+)-Tramadolhydrochlorid) befüllt.

| **Zusammensetzung pro Kapsel:** | |
|---|---|
| Pellets enthaltend das Racemat des Tramadols: Racemisches Tramadolhydrochlorid | 212,0 mg 100,0 mg |
| Mikrokristalline Cellulose (Avicel PH 105® von FMC) | 42,7 mg |
| Niedrig substituierte Hydroxypropylcellulose (I-HPC LH 31® von ShinEtsu) | 40,8 mg |
| | |
| Hydroxypropylmethylcellulose | 4,8 mg |
| Opadry OY 29020 clear® (Colorcon) | |
| Talkum | 1,6 mg |
| Ethylcellulose | 22,1 mg |
| Surelease E-7-7050® (Colorcon) | |
| | |
| Pellets enthaltend das (+)-Enantiomer des Tramadols | 47,0 mg |
| | |
| (+)-Tramadolhydrochlorid | 25,0 mg |
| Mikrokristalline Cellulose (Avicel PH 105® von FMC) | 10,5 mg |
| Niedrig substituierte Hydroxypropylcellulose (I-HPC LH 31® von ShinEtsu) | 10,0 mg |
| Hydroxypropylmethylcellulose | 1,2 mg |
| Opadry OY 29020 clear® (Colorcon) | |
| Talkum | 0,3 mg |

Das Freisetzungsprofil wurde gemäß der oben angegebenen Methode in der Drehkörbchenapparatur bestimmt und ist in der nachfolgenden Tabelle 1 wiedergegeben.

**Tabelle 1:**

| Zeit in Minuten | Freigesetzter Anteil an (+)-Tramadol in mg | Freigesetzter Anteil an (-)-Tramadol in mg |
|---|---|---|
| 30 | 25 | 0 |
| 120 | 28 | 4 |
| 240 | 40 | 15 |
| 360 | 55 | 31 |
| 480 | 65 | 41 |
| 600 | 74 | 49 |

### Beispiel 2:

Pellets enthaltend das Racemat des Tramadols und Pellets enthaltend das (+)-Enantiomer des Tramadols der nachstehend angegebenen Zusammensetzungen wurden analog zu Beispiel 1 hergestellt und überzogen.

Auf einer geeigneten Steckkapselmaschine wurden dann Hartgelatinekapseln der Größe 0 mit 212 mg der retardierten Pellets enthaltend racemisches Tramadol (entsprechend 100 mg racemischem Tramadolhydrochlorid) und 94 mg der unretardierten Pellets enthaltend das (+)-Enantiomer des Tramadols (entsprechend 50 mg (+)-Tramadolhydrochlorid) befüllt.

| **Zusammensetzung pro Kapsel:** | |
|---|---|
| Pellets enthaltend das Racemat des Tramadols: | 212,0 mg |
| | |
| Racemisches Tramadolhydrochlorid | 100,0 mg |
| Mikrokristalline Cellulose (Avicel PH 105® von FMC) | 42,7 mg |
| Niedrig substituierte Hydroxypropylcellulose (I-HPC LH 32® von ShinEtsu) | 40,8 mg |
| Hydroxypropylmethylcellulose | 4,8 mg |
| Opadry OY 29020 clear® (Colorcon) | |
| Talkum | 1,6 mg |
| Ethylcellulose | 22,1 mg |
| Surelease E-7-7050® (Colorcon) | |
| | |
| Pellets enthaltend das (+)-Enantiomer des Tramadols | 94,0 mg |
| | |
| (+)-Tramadolhydrochlorid | 50,0 mg |
| Mikrokristalline Cellulose (Avicel PH 105® von FMC) | 21,0 mg |
| Niedrig substituierte Hydroxypropylcellulose (I-HPC LH 32® von ShinEtsu) | 20,0 mg |
| Hydroxypropylmethylcellulose | 2,4 mg |
| Opadry OY 29020 clear® (Colorcon) | |
| Talkum | 0,6 mg |

Das Freisetzungsprofil wurde gemäß der oben angegebenen Methode in der Drehkörbchenapparatur bestimmt und ist in der nachfolgenden Tabelle 2 wiedergegeben.

**Tabelle 2:**

| Zeit in Minuten | Freigesetzter Anteil an (+)-Tramadol in mg | Freigesetzter Anteil an (-)-Tramadol in mg |
|---|---|---|
| 30 | 51 | 0 |
| 120 | 52 | 3 |
| 240 | 64 | 14 |
| 360 | 81 | 32 |
| 480 | 92 | 42 |
| 600 | 99 | 50 |

### Beispiel 3:

Racemisches Tramadolhydrochlorid wurde mit mikrokristalliner Cellulose, Hydroxypropylmethylcellulose, hochdispersem Siliciumdioxid und Magnesiumstearat in einem Kubus-Mischer homogen gemischt.

(+)-Tramadolhydrochlorid wurde mit mikrokristalliner Cellulose, hochdispersem Siliciumdioxid und Magnesiumstearat in einem Kubus-Mischer homogen gemischt. Die beiden Mischungen wurden anschließend auf einer Tablettenpresse (Korsch EK0) Exzenter zu Zweischichttabletten mit einem mittleren Durchmesser von 12 mm verpreßt. Hierzu wurden zunächst die 250 mg Pulvermischung der ersten Schicht in die Matrize eingefüllt, per Handbetrieb vorgepreßt und nach Zugabe der 100 mg Mischung der zweiten Schicht die Tabletten endverpreßt.

### Zusammensetzung einer Zweischichttablette:

| 1. Schicht | |
|---|---|
| Racemisches Tramadolhydrochlorid | 100,0 mg |
| Mikrokristalline Cellulose (Avicel PH 101® von FMC) | 82,0 mg |
| Hydroxypropylmethylcellulose Typ 2910, 100 000 mPas (Metolose 90 SH 100 000® ShinEtsu) | 63,0 mg |
| Hochdisperses Siliciumdioxid (Aerosil®, Degussa) | 2,5 mg |
| Magnesiumstearat | 2,5 mg |
| Gesamt (1. Schicht) | 250 mg |

| 2. Schicht | |
|---|---|
| (+)-Tramadolhydrochlorid | 50,0 mg |
| Mikrokristalline Cellulose (Avicel PH 101® von FMC) | 48,0 mg |
| Hochdisperses Siliciumdioxid (Aerosil®, Degussa) | 1,0 mg |
| Magnesiumstearat | 1,0 mg |
| Gesamt (2. Schicht) | 100 mg |
| Gesamt (Zweischichttablette) | 350 mg |

Das Freisetzungsprofil wurde gemäß der oben angegebenen Methode in der Drehkörbchenapparatur bestimmt und ist in der nachfolgenden Tabelle 3 wiedergegeben. Abweichend von der angegebenen Methode wurde 10 Stunden in künstlichem Darmsaft geprüft.

**Tabelle 3:**

| Zeit in Minuten | Freigesetzter Anteil an (+)-Tramadol in mg | Freigesetzter Anteil an (-)-Tramadol in mg |
|---|---|---|
| 30 | 57 | 10 |
| 60 | 67 | 17 |
| 120 | 79 | 27 |
| 180 | 83 | 33 |
| 240 | 85 | 37 |
| 360 | 92 | 42 |
| 480 | 96 | 44 |
| 600 | 97 | 48 |
| 720 | 97 | 49 |

## Patentansprüche

1. Arzneimittel enthaltend das Racemat des Tramadols in retardierter Form und das (+)-Enantiomer des Tramadols in unretardierter Form.

2. Arzneimittel nach Anspruch 1, **dadurch gekennzeichnet, daß** wenigstens eine der beiden Wirkstoffkomponenten in Form wenigstens eines ihrer entsprechenden physiologisch verträglichen Salze vorliegt.

3. Arzneimittel gemäß Anspruch 2, **dadurch gekennzeichnet, daß** das physiologisch verträgliche Salz ausgewählt ist aus der Gruppe Chlorid, Bromid, Sulfat, Sulfonat, Phosphat, Tartrat, Teoclat, Embonat, Formiat, Acetat, Propionat, Benzoat, Oxalat, Succinat, Citrat, Diclofenacat, Naproxenat, Salicylat, Acetylsalicylat, Glutamat, Fumarat, Aspartat, Glutarat, Stearat, Butyrat, Malonat, Lactat, Mesylat, Saccharinat, Cyclamat und Acesulfamat, vorzugsweise aus der Gruppe Chlorid, Sulfat, Saccharinat, Teoclat, Embonat, Diclofenacat, Naproxenat und Salicylat, besonders bevorzugt das Chlorid ist.

4. Arzneimittel gemäß einem der Ansprüche 1 bis 3, **dadurch gekennzeichnet, daß** es 10 bis 75 Gew.-% (+)-Tramadol und 90 bis 25 Gew.-% retardiertes racemisches Tramadol, vorzugsweise 20 bis 50 Gew.-% (+) Tramadol und 80 bis 50 Gew.-% retardiertes racemisches Tramadol, jeweils berechnet als freier Wirkstoff und bezogen auf die Gesamtmenge der Wirkstoffe, enthält.

5. Arzneimittel gemäß einem der Ansprüche 1 bis 4 zur oralen oder parenteralen, vorzugsweise zur oralen Applikation.

6. Arzneimittel gemäß einem der Ansprüche 1 bis 5, **dadurch gekennzeichnet, daß** es die beiden Wirkstoffkomponenten in jeweils separiert voneinander formulierten Untereinheiten aufweist.

7. Arzneimittel gemäß Anspruch 6, **dadurch gekennzeichnet, daß** die Untereinheiten verschiedene Schichten einer Mehrschichttablette, vorzugsweise die Schichten einer Zweischichttablette sind.

8. Arzneimittel gemäß Anspruch 6, **dadurch gekennzeichnet, daß** die Untereinheiten in multipartikulärer Form, vorzugsweise in Form von Mikrotabletten, Mikrokapseln, Granulaten, Wirkstoffkristallen oder Pellets vorliegen.

9. Arzneimittel gemäß Anspruch 7 oder 8, **dadurch gekennzeichnet, daß** es das Racemat in retardierten Untereinheiten aufweist, die mit einem Überzug enthaltend das (+)-Enantiomer ausgerüstet sind.

10. Arzneimittel gemäß einem der Ansprüche 1 bis 9, **dadurch gekennzeichnet, daß** die Retardierung des Racemates durch einen retardierenden Überzug, durch die Einbettung in eine retardierende Matrix, durch Anbindung an ein lonenaustauscherharz oder eine Kombination aus wenigstens zwei dieser Retardierungsmethoden erfolgt ist.

11. Arzneimittel gemäß Anspruch 10, **dadurch gekennzeichnet, daß** der retardierende Überzug auf einem wasserunlöslichen gegebenenfalls modifizierten natürlichen oder synthetischen Polymeren oder auf einem natürlichen, halbsynthetischen oder synthetischen Wachs oder Fett oder Fettalkohol oder einem Gemisch aus wenigstens zwei dieser Komponenten basiert.

12. Arzneimittel gemäß Anspruch 11, **dadurch gekennzeichnet, daß** als wasserunlösliche Polymere Poly(meth)acrylate, bevorzugt Poly(C₁₋₄)-Alkyl(meth)acrylate, Poly(C₁₋₄)-dialkylamino-(C₁₋₄)-alkyl(meth)acrylate und/oder deren Copolymere, besonders bevorzugt Ethylacrylat/Methylmethacrylat-Copolymere mit einem molaren Verhältnis der Monomeren von 2 : 1, Ethylacrylat/Methylmethacrylat/Trimethylammoniumethylmethacrylatchlorid-Copolymere mit einem molaren Verhältnis der Monomeren von 1 : 2 : 0,1, Ethylacrylat/Methylmethacrylat/Trimethylammoniumethylmethacrylatchlorid-Copolymere mit einem molaren Verhältnis der Monomeren von 1 : 2 : 0,2 oder ein Gemisch aus wenigstens zwei dieser vorstehend genannten Polymeren als Überzugsmaterial vorliegen.

13. Arzneimittel gemäß Anspruch 11, **dadurch gekennzeichnet, daß** als wasserunlösliche Polymere Cellulosederivate, vorzugsweise Alkylcellulose, besonders bevorzugt Ethylcellulose, oder Celluloseester, vorzugsweise Celluloseacetat als Überzugsmaterial vorliegen.

14. Arzneimittel gemäß Anspruch 12 oder 13, **dadurch gekennzeichnet, daß** die Polymere aus wäßrigem Medium, vorzugsweise aus wäßriger Latex oder Pseudolatex-Dispersionen aufgetragen worden sind.

15. Arzneimittel gemäß Anspruch 11, **dadurch gekennzeichnet, daß** als Überzugs-Polymere ein Gemisch aus Polyvinylacetat und Polyvinylpyrollidon, vorzugsweise in Form wäßriger Pseudolatex-Dispersionen eingesetzt worden ist.

16. Arzneimittel gemäß Anspruch 11, **dadurch gekennzeichnet, daß** als Wachs Carnaubawachs, Bienenwachs, Glycerinmonostearat, Glycerinmonobehenat, Glycerinditripalmitostearat, mikrokristallines Wachs oder ein Gemisch aus wenigstens zwei dieser Wachse als Überzugsmaterial vorliegt.

17. Arzneimittel gemäß einem der Ansprüche 11 bis 16, **dadurch gekennzeichnet, daß** die Polymere in Kombination mit wenigstens einem üblichen Weichmacher eingesetzt worden sind.

18. Arzneimittel gemäß Anspruch 17, **dadurch gekennzeichnet, daß** als Weichmacher lipophile Diester aus aliphatischen oder aromatischen Dicarbonsäuren mit C₆-C₄₀ und aliphatischen Alkoholen mit C₁-C₈, hydrophile oder lipophile Ester der Zitronensäure, Polyalkylenglykole, Ester des Glycerins, acetylierte Mono- und/oder Diglyceride, mittelkettige Triglyceride, Ölsäure oder ein Gemisch aus wenigstens zwei dieser Weichmacher eingesetzt worden sind.

19. Arzneimittel gemäß einem der Ansprüche 17 oder 18, **dadurch gekennzeichnet, daß** der Weichmacher in Mengen von 5 bis 50 Gew-%., vorzugsweise 10 bis 40 Gew.-%, besonders bevorzugt 10 bis 30 Gew-%, bezogen auf das polymere Überzugsmaterial, eingesetzt worden ist.

20. Arzneimittel gemäß Anspruch 10, **dadurch gekennzeichnet, daß** die retardierende Matrix auf einem hydrophilen Matrixmaterial, vorzugsweise hydrophilen Polymeren, besonders bevorzugt auf Celluloseethern, Celluloseestern und/oder Acrylharzen, ganz besonders bevorzugt auf Ethylcellulose, Hydroxypropylmethylcellulose, Hydroxypropylcellulose, Hydroxymethylcellulose, Poly(meth)acrylsäure und/oder deren Salzen, Amiden und/oder Estern oder auf einem Gemisch aus wenigstens zwei dieser hydrophilen Matrixmaterialien basiert.

21. Arzneimittel gemäß Anspruch 10, **dadurch gekennzeichnet, daß** die Matrix auf einem hydrophoben Matrixmaterial, vorzugsweise hydrophoben Polymeren, Wachsen, Fetten, langkettigen Fettsäuren, Fettalkoholen oder entsprechenden Estern oder Ethern oder deren Gemischen, besonders bevorzugt auf Mono- oder Diglyceriden von C₁₂-C₃₀-Fettsäuren und/oder C₁₂-C₃₀-Fettalkoholen und/oder Wachsen oder auf einem Gemisch aus wenigstens zwei dieser hydrophoben Matrixmaterialien basiert.

22. Arzneimittel gemäß einem der Ansprüche 1 bis 21, **dadurch gekennzeichnet, daß** es wenigstens einen Schutzüberzug aufweist.

23. Arzneimittel gemäß Anspruch 22, **dadurch gekennzeichnet, daß** der Schutzüberzug der Geschmacksverbesserung dient.

24. Arzneimittel gemäß Anspruch 22, **dadurch gekennzeichnet, daß** der Schutzüberzug magensaftresistent ist.

25. Arzneimittel gemäß Anspruch 24, **dadurch gekennzeichnet, daß** der magensaftresistente Überzug aus Methacrylsäure/Methylmethacrylat-Copolymeren mit einem molaren Verhältnis der Monomeren von 1 : 1, Methacrylsäure/Methylmethacrylat-Copolymeren mit einem molaren Verhältnis der Monomeren von 1 : 2, Methacrylsäure/Ethylacrylat-Copolymeren mit einem molaren Verhältnis der Monomeren von 1 : 1, Methacrylsäure/Methylacrylat/Methylmethacrylat mit einem molaren Verhältnis der Monomeren von 7 : 3 : 1, Schellack, Hydroxypropylmethylcelluloseacetatsuccinat, Celluloseacetatphthalat oder aus einer Mischung aus wenigstens zwei dieser Komponenten, gegebenenfalls auch in Kombination mit Poly(meth)acrylaten besteht.

26. Arzneimittel gemäß einem der Ansprüche 1 bis 25 zur Bekämpfung von Schmerzen.

27. Arzneimittel gemäß Anspruch 26 zur Bekämpfung von akuten Schmerzen.

28. Arzneimittel gemäß Anspruch 26 zur Bekämpfung von chronischen Schmerzen.

29. Arzneimittel gemäß einem der Ansprüche 1 bis 25 zur Behandlung von Harninkontinenz.

30. Arzneimittel gemäß einem der Ansprüche 1 bis 25 zur Behandlung von Husten.

31. Arzneimittel gemäß einem der Ansprüche 1 bis 25 zur Behandlung von Depressionen.

32. Arzneimittel gemäß einem der Ansprüche 1 bis 25 zur Behandlung Diarrhoe.

33. Arzneimittel gemäß einem der Ansprüche 1 bis 25 zur Behandlung von seelischen Erkrankungen.

## Claims

1. Medicament comprising the racemate of tramadol in slow-release form and the (+) enantiomer of tramadol in immediate-release form.

2. Medicament according to Claim 1, **characterized in that** at least one of the two active ingredient components is present in the form of at least one of its appropriate physiologically tolerated salts.

3. Medicament according to Claim 2, **characterized in that** the physiologically tolerated salt is selected from the group of chloride, bromide, sulphate, sulphonate, phosphate, tartrate, teoclate, embonate, formate, acetate, propionate, benzoate, oxalate, succinate, citrate, diclofenacate, naproxenate, salicylate, acetylsalicylate, glutamate, fumarate, aspartate, glutarate, stearate, butyrate, malonate, lactate, mesylate, saccharinate, cyclamate and acesulphamate, preferably from the group of chloride, sulphate, saccharinate, teoclate, embonate, diclofenacate, naproxenate and salicylate, particularly preferably is the chloride.

4. Medicament according to any of Claims 1 to 3, **characterized in that** it contains from 10 to 75% by weight of (+)-tramadol and from 90 to 25% by weight of slow-release racemic tramadol, preferably from 20 to 50% by weight of (+)-tramadol and from 80 to 50% by weight of slow-release racemic tramadol, in each case calculated as free active ingredient and based on the total amount of active ingredients.

5. Medicament according to any of Claims 1 to 4 for oral or parenteral, preferably for oral administration.

6. Medicament according to any of Claims 1 to 5, **characterized in that** it includes the two active ingredient components in subunits each formulated separately from one another.

7. Medicament according to Claim 6, **characterized in that** the subunits are different layers of a multilayer tablet, preferably the layers of a bilayer tablet.

8. Medicament according to Claim 6, **characterized in that** the subunits are present in multiparticulate form, preferably in the form of microtablets, microcapsules, granules, active ingredient crystals or pellets.

9. Medicament according to Claim 7 or 8, **characterized in that** it includes the racemate in slow-release subunits which are provided with a coating containing the (+) enantiomer.

10. Medicament according to any of Claims 1 to 9, **characterized in that** the slowing of release of the racemate is effected by a release-slowing coating, by embedding in a release-slowing matrix, by attachment to an ion-exchange resin or a combination of at least two of these release-slowing methods.

11. Medicament according to Claim 10, **characterized in that** the release-slowing coating is based on a water-insoluble optionally modified natural or synthetic polymer or on a natural, semisynthetic or synthetic wax or fat or fatty alcohol or a mixture of at least two of these components.

12. Medicament according to Claim 11, **characterized in that** water-insoluble polymers present are poly(meth)acrylates, preferably poly(C₁₋₄)-alkyl (meth)acrylates, poly(C₁₋₄)dialkylamino-(C₁₋₄)-alkyl (meth)acrylates and/or copolymers thereof, particularly preferably ethyl acrylate/methyl methacrylate copolymers with a monomer molar ratio of 2:1, ethyl acrylate/methyl methacrylate/trimethylammoniumethyl methacrylate chloride copolymers with a monomer molar ratio of 1:2:0.1, ethyl acrylate/methyl methacrylate/trimethyl-ammoniumethyl methacrylate chloride copolymers with a monomer molar ratio of 1:2:0.2 or a mixture of at least two of these aforementioned polymers as coating material.

13. Medicament according to Claim 11, **characterized in that** water-insoluble polymers present are cellulose derivatives, preferably alkylcellulose, particularly preferably ethylcellulose or cellulose esters, preferably cellulose acetate, as coating material.

14. Medicament according to Claim 12 or 13, **characterized in that** the polymers have been applied from aqueous medium, preferably from aqueous latex or pseudolatex dispersions.

15. Medicament according to Claim 11, **characterized in that** a mixture of polyvinyl acetate and polyvinylpyrrolidone, preferably in the form of aqueous pseudolatex dispersions, has been employed as coating polymer.

16. Medicament according to Claim 11, **characterized in that** the wax present as coating material is carnauba wax, beeswax, glycerol monostearate, glycerol monobehenate, glycerol ditripalmitostearate, microcrystalline wax or a mixture of at least two of these waxes.

17. Medicament according to any of Claims 11 to 16, **characterized in that** the polymers have been employed in combination with at least one conventional plasticizer.

18. Medicament according to Claim 17, **characterized in that** the plasticizers which have been employed are lipophilic diesters of aliphatic or aromatic dicarboxylic acids with C₆-C₄₀ and aliphatic alcohols with C₁-C₈, hydrophilic or lipophilic esters of citric acid, polyalkylene glycols, esters of glycerol, acetylated mono- and/or diglycerides, medium chain-length triglycerides, oleic acid or a mixture of at least two of these plasticizers.

19. Medicament according to either of Claims 17 or 18, **characterized in that** the plasticizer has been employed in amounts of from 5 to 50% by weight, preferably 10 to 40% by weight, particularly preferably 10 to 30% by weight, based on the polymeric coating material.

20. Medicament according to Claim 10, **characterized in that** the release-slowing matrix is based on a hydrophilic matrix material, preferably hydrophilic polymers, particularly preferably on cellulose ethers, cellulose esters and/or acrylic resins, very particularly preferably on ethylcellulose, hydroxypropylmethylcellulose, hydroxypropylcellulose, hydoxymethylcellulose, poly(meth)acrylic acid and/or salts, amides and/or esters thereof or on a mixture of at least two of these hydrophilic matrix materials.

21. Medicament according to Claim 10, **characterized in that** the matrix is based on a hydrophobic matrix material, preferably hydrophobic polymers, waxes, fats, long-chain fatty acids, fatty alcohols or corresponding esters or ethers or mixtures thereof, particularly preferably on mono- or diglycerides of C₁₂-C₃₀ fatty acids and/or C₁₂-C₃₀ fatty alcohols and/or waxes or on a mixture of at least two of these hydrophobic matrix materials.

22. Medicament according to any of Claims 1 to 21, **characterized in that** it includes at least one protective coating.

23. Medicament according to Claim 22, **characterized in that** the protectve coating serves to improve the taste.

24. Medicament according to Claim 22, **characterized in that** the protective coating is resistant to gastric fluid.

25. Medicament according to Claim 24, **characterized in that** the coating resistant to gastric fluid consists of methacrylic acid/methyl methacrylate copolymers with a monomer molar ratio of 1:1, methacrylic acid/methyl methacrylate copolymers with a monomer molar ratio of 1:2, methacrylic acid/ethyl acrylate copolymers with a monomer molar ratio of 1:1, methacrylic acid/methyl acrylate/methyl methacrylate with a monomer molar ratio of 7:3:1, shellac, hydroxypropylmethylcellulose acetate succinate, cellulose acetate phthalate or of a mixture of at least two of these components, where appropriate also in combination with poly(meth)acrylates.

26. Medicament according to any of Claims 1 to 25 for controlling pain.

27. Medicament according to Claim 26 for controlling acute pain.

28. Medicament according to Claim 26 for controlling chronic pain.

29. Medicament according to any of Claims 1 to 25 for treating urinary incontinence.

30. Medicament according to any of Claims 1 to 25 for treating cough.

31. Medicament according to any of Claims 1 to 25 for treating depression.

32. Medicament according to any of Claims 1 to 25 for treating diarrhoea.

33. Medicament according to any of Claims 1 to 25 for treating mental disorders.

## Revendications

1. Médicament contenant le mélange racémique du tramadol sous forme retardée et l'énantiomère-(+) du tramadol sous forme non retardée.

2. Médicament selon la revendication 1, **caractérisé en ce qu'**au moins un des deux composants d'agent actif est présent sous la forme d'au moins un de leurs sels correspondants, compatibles du point de vue physiologique.

3. Médicament selon la revendication 2, **caractérisé en ce que** le sel compatible du point du vue physiologique est sélectionné parmi le groupe chlorure, bromure, sulfate, sulfonate, phosphate, tartrate, téoclate, embonate, formiate, acétate, propionate, benzoate, oxalate, succinate, citrate, diclofénacate, naproxénate, salicylate, acétylsalicylate, glutamate, fumarate, aspartate, glutarate, stéarate, butyrate, malonate, lactate, mésylate, saccharinate, cyclamate et acésulfamate, de préférence, du groupe chlorure, sulfate, saccharinate, téoclate, embonate, diclofénacate, naproxénate et salicylate, en particulier, de préférence, **en ce que** le sel est le chlorure.

4. Médicament selon l'une quelconque des revendications 1 à 3, **caractérisé en ce qu'**il contient de 10 à 75% en poids de tramadol-(+) et de 90 à 25 % en poids de tramadol racémique retardé, de préférence, de 20 à 50% en poids de tramadol(+) et de 80 à 50% en poids de tramadol racémique retardé, le calcul étant effectué à chaque fois en tant qu'agent actif libre et se rapportant à la quantité totale des agents actifs.

5. Médicament selon l'une quelconque des revendications 1 à 4 en vue de l'application orale ou parentérale, de préférence, en vue de l'application orale.

6. Médicament selon l'une quelconque des revendications 1 à 5, **caractérisé en ce qu'**il présente les deux composants d'agent actif dans des sous-unités formulées à chaque fois de manière séparée l'une de l'autre.

7. Médicament selon la revendication 6, **caractérisé en ce que** les sous-unités sont diverses couches d'un comprimé à plusieurs couches, de préférence, les couches d'un comprimé à deux couches.

8. Médicament selon la revendication 6, **caractérisé en ce que** les sous-unités sont présentes sous une forme multiparticulaire, de préférence, sous la forme de micro comprimés, de micro capsules, de granulés, de cristaux d'agent actif ou de pastilles.

9. Médicament selon la revendication 7 ou 8, **caractérisé en ce qu'**il présente le mélange racémique dans des sous-unités retardées, qui sont pourvues d'un revêtement contenant l'énantiomère-(+).

10. Médicament selon l'une quelconque des revendications 1 à 9, **caractérisé en ce que** le retardement du mélange racémique se fait par l'intermédiaire d'un revêtement retardateur, par le noyage dans une matrice retardatrice, par la fixation sur une résine échangeuse d'ions ou par l'intermédiaire d'une combinaison d'au moins deux de ces procédés de retardement.

11. Médicament selon la revendication 10, **caractérisé en ce que** le revêtement retardateur est basé sur un polymère naturel ou synthétique, non hydrosoluble, le cas échéant, modifié ou sur une cire ou une graisse ou un alcool gras naturel, semi-synthétique ou synthétique ou sur un mélange d'au moins deux de ces composants.

12. Médicament selon la revendication 11, **caractérisé en ce que** sont présents, en tant que matériau de revêtement, sous la forme de polymères non hydrosolubles, des poly(méth)acrylates, de préférence, des (méth)acrylates de poly(C₁₋₄)-alkyle, des (méth) acrylates de poly(C₁₋₄)-dialkylamino(C₁₋₄)-alkyle et/ou leurs copolymères, en particulier, de préférence, de copolymères acrylate d'éthyle/méthacrylate de méthyle, ayant un rapport molaire des monomères de 2 : 1, de copolymères acrylate d'éthyle/méthacrylate de méthyle/chlorure de méthacrylate de triméthylammoniuméthyle, ayant un rapport molaire des monomères de 1 : 2 : 0,1, de copolymères acrylate d'éthyle/méthacrylate de méthyle/chlorure de méthacrylate de triméthylammoniuméthyle, ayant un rapport molaire des monomères de 1 : 2 : 0,2 ou un mélange d'au moins deux de ces polymères susnommés.

13. Médicament selon la revendication 11, **caractérisé en ce que** sont présents, en tant que matériau de revêtement, sous la forme de polymères non hydrosolubles, des dérivés de la cellulose, de préférence, des celluloses alkyles, en particulier, de préférence, l'éthylcellulose ou des esters de cellulose, de préférence, l'acétate de cellulose.

14. Médicament selon la revendication 12 ou 13, **caractérisé en ce que** les polymères ont été appliqués à partir d'un milieu aqueux, de préférence, à partir d'un latex aqueux ou de dispersions de pseudolatex.

15. Médicament selon la revendication 11, **caractérisé en ce que** l'on a utilisé, en tant que polymères de revêtement, un mélange d'acétate de polyvinyle et de polyvinylpyrollidone, de préférence, sous la forme de dispersions aqueuses de pseudolatex.

16. Médicament selon la revendication 11, **caractérisé en ce que** sont présents, en tant que matériau de revêtement, sous la forme de cire, la cire de Carnauba, la cire d'abeilles, le mono stéarate de glycérine, le monobéhénate de glycérine, le ditripalmitostéarate de glycérine, la cire micro cristalline ou un mélange d'au moins deux de ces cires.

17. Médicament selon l'une quelconque des revendications 11 à 16, **caractérisé en ce que** les polymères ont été utilisés en combinaison avec au moins un plastifiant usuel.

18. Médicament selon la revendication 17, **caractérisé en ce que** l'on a utilisé en tant que plastifiant des diesters lipophiles d'acides dicarboxyliques aliphatiques ou aromatiques en C₆-C₄₀ et des alcools aliphatiques en C₁-C₈, des esters hydrophiles ou lipophiles de l'acide citrique, des polyalkylèneglycols, des esters de la glycérine, des monoglycérides et/ou des diglycérides acétylés, des triglycérides à longueur de chaîne moyenne, l'acide oléique ou un mélange d'au moins deux de ces plastifiants.

19. Médicament selon l'une quelconque des revendications 17 ou 18, **caractérisé en ce que** le plastifiant a été utilisé dans des quantités de 5 à 50% en poids, de préférence, de 10 à 40% en poids, en particulier, de préférence, de 10 à 30% en poids, par rapport au matériau de revêtement polymère.

20. Médicament selon la revendication 10, **caractérisé en ce que** la matrice retardatrice est basée sur un matériau de matrice hydrophile, de préférence, sur des polymères hydrophiles, en particulier, de préférence, sur des éthers de cellulose, sur des esters de cellulose et/ou sur des résines acryliques, tout particulièrement, de préférence, sur l'éthylcellulose, sur l'hydroxypropylméthylcellulose, sur l'hydroxypropylcellulose, sur l'hydroxyméthylcellulose, sur l'acide poly(méth)acrylique et/ou sur leurs sels, sur leurs amides et/ou sur leurs esters ou sur un mélange d'au moins deux de ces matériaux de matrice hydrophiles.

21. Médicament selon la revendication 10, **caractérisé en ce que** la matrice est basée sur un matériau de matrice hydrophobe, de préférence, sur des polymères hydrophobes, sur des cires, sur des graisses, sur des acides gras à longue chaîne, sur des alcools gras ou sur leurs esters ou éthers correspondants ou sur leurs mélanges, en particulier, de préférence, sur des monoglycérides et/ou des diglycérides d'acides gras en C₁₂₋C₃₀ et/ou des alcools gras en C₁₂-C₃₀ et/ou des cires ou sur un mélange d'au moins deux de ces matériaux de matrice hydrophobes.

22. Médicament selon l'une quelconque des revendications 1 à 21, **caractérisé en ce qu'**il présente au moins un revêtement de protection.

23. Médicament selon la revendication 22, **caractérisé en ce que** le revêtement de protection sert à l'amélioration du goût.

24. Médicament selon la revendication 22, **caractérisé en ce que** le revêtement de protection est résistant aux sécrétions de l'estomac.

25. Médicament selon la revendication 24, **caractérisé en ce que** le revêtement résistant aux sécrétions de l'estomac se compose de copolymères acide méthacrylique/méthacrylate de méthyle, ayant un rapport molaire des monomères de 1 : 1, de copolymères acide méthacrylique/méthacrylate de méthyle, ayant un rapport molaire des monomères de 1 : 2, des copolymères acide méthacrylique/acrylate d'éthyle, ayant un rapport molaire des monomères de 1 : 1, des copolymères acide méthacrylique/acrylate de méthyle/méthacrylate de méthyle, ayant un rapport molaire des monomères de 7 : 3 : 1, de gomme raffinée, d'acétate-succinate d'hydroxypropylméthylcellulose, d'acétate-phtalate de cellulose, ou d'un mélange d'au moins deux de ces composants, le cas échéant, en combinaison également avec des poly(méth)acrylates.

26. Médicament selon l'une quelconque des revendications 1 à 25 en vue de la lutte contre les douleurs.

27. Médicament selon la revendication 26 en vue de la lutte contre les douleurs aiguës.

28. Médicament selon la revendication 26 en vue de la lutte contre les douleurs chroniques.

29. Médicament selon l'une quelconque des revendications 1 à 25 en vue du traitement de l'incontinence urinaire.

30. Médicament selon l'une quelconque des revendications 1 à 25 en vue du traitement de la toux.

31. Médicament selon l'une quelconque des revendications 1 à 25 en vue du traitement des dépressions.

32. Médicament selon l'une quelconque des revendications 1 à 25 en vue du traitement de la diarrhée.

33. Médicament selon l'une quelconque des revendications 1 à 25 en vue du traitement des maladies psychiques.
